# EUROPÄISCHE PATENTSCHRIFT

(11) **EP 3 215 104 B1**
(45) Veröffentlichungstag und Bekanntmachung des Hinweises auf die Patenterteilung: **04.12.2019**
(21) Anmeldenummer: 15771927.9
(22) Anmeldetag: 29.09.2015
(51) Int. Cl.: A61K 8/25, A61K 8/34, A61Q 5/06, A61K 8/81

(54) **MITTEL ZUR TEMPORÄREN VERFORMUNG KERATINHALTIGER FASERN ENTHALTEND EIN HYDROPHOB MODIFIZIERTES METALLOXIDPULVER, EIN FILMBILDENDES POLYMER UND ETHANOL**
AGENTS FOR THE TEMPORARY SHAPING OF KERATIN-CONTAINING FIBERS, CONTAINING A HYDROPHOBICALLY MODIFIED METAL OXIDE POWDER, A FILM-FORMING POLYMER AND ETHANOL
PRODUITS DE MISE EN FORME TEMPORAIRE DE FIBRES KÉRATINIQUES, CONTENANT UNE POUDRE D'OXYDE MÉTALLIQUE MODIFIÉE DE FAÇON HYDROPHOBE, UN POLYMÈRE FILMOGÈNE ET DE L'ÉTHANOL

(30) Priorität: 07.11.2014 DE 102014222802
(43) Veröffentlichungstag der Anmeldung: 13.09.2017
(73) Patentinhaber: Henkel AG & Co. KGaA, 40589 Düsseldorf (DE)
(72) Erfinder: KNAPPE, Thorsten, 22869 Schenefeld (DE); HEINSOHN, Ulrike, 22529 Hamburg (DE)
(86) Internationale Anmeldenummer: PCT/EP2015/072336
(87) Internationale Veröffentlichungsnummer: WO 2016/071044

(56) Entgegenhaltungen:
- EP-A1- 1 942 988
- EP-A1- 2 570 190
- WO-A2-2009/047100
- WO-A2-2013/072118
- DE-T2- 68 915 171
- US-A1- 2013 309 282

## Beschreibung

Die Anmeldung betrifft das technische Fachgebiet der temporären Umformung keratinhaltiger Fasern, insbesondere menschlicher Haare. Gegenstand der Anmeldung sind kosmetische Mittel, enthaltend mindestens ein hydrophobiertes Metalloxidpulver, filmbildendes Polymer und Ethanol, sowie kosmetische Produkte, umfassend ein Abgabevorrichtung mit Sprühventil sowie die zuvor genannten kosmetischen Mittel. Darüber hinaus sind Gegenstand der vorliegenden Anmeldung die Verwendung dieser kosmetischen Mittel und Produkte zur temporären Umformung keratinhaltiger Fasern sowie entsprechende Anwendungsverfahren.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, welche sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, welche einer permanenten oder temporären Formgebung der Haare dienen, eine wichtige Rolle. Während bei der permanenten Umformung die chemische Struktur der keratinhaltigen Faser durch Reduktion und Oxidation modifiziert wird, finden solche Modifikationen der chemischen Struktur bei der temporären Umformung nicht statt. Entsprechende Mittel zur temporären Verformung enthalten als festigenden Wirkstoff üblicherweise synthetische Polymere und/oder Wachse.

Die wichtigste Eigenschaft eines Mittels zur temporären Verformung keratinischer Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinischen Fasern um menschliche Haare, spricht man auch von starkem Frisurenhalt oder von hohen Haltegrad des Stylingmittels. Der Frisurenhalt wird im Wesentlichen durch die Art und Menge der eingesetzten festigenden Wirkstoffe bestimmt, wobei jedoch auch ein Einfluss der weiteren Bestandteile des Stylingmittels sowie der Applikationsform gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. des versprühten Aerosols oder Non-Aerosols, und Eigenschaften, welche die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde im Stand der Technik eine Vielzahl von synthetischen Polymeren als festigende Wirkstoffe entwickelt, welche in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, welcher einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken bzw. Rückständen, welche sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden.

Mittel zur Unterstützung der temporären Umformung keratinhaltiger Fasern können beispielsweise als Haarspray, Haarwachs, Haargel oder Haarschaum konfektioniert werden. Insbesondere die Applikation mittels einer Sprühvorrichtung in Form eines Sprays erfreut sich hoher Beliebtheit. Jedoch erfordert die Applikation mittels einer Sprühvorrichtung, je nach Beschaffenheit des Stylingmittels, zusätzliche Maßnahmen. So ist beispielsweise die Sprühapplikation verdickter Haargele im Stand der Technik bislang nur unter Verwendung einer bestimmten Kombination von Verdickungsmittel und Stylingpolymer möglich, da nur auf diese Weise eine ausreichende Vernebelung des Haargels mittels der Sprühapplikation gewährleistet werden kann. Die im Stand der Technik verwendete Kombination aus einem Carbomer und PVP/VA-Stylingpolymeren lässt sich zwar ausreichend vernebeln, weist jedoch keinen zufriedenstellenden Langzeithalt und Volumeneffekt auf und entspricht daher nicht mehr dem Verbraucherbedürfnis.

US2013/309282 offenbart Mittel zur temporären Verformung keratinischer Fasern, umfassend Silica Silylate, filmbildenden Polymere, Ethanol und Wasser.

Aufgabe der vorliegenden Erfindung war es daher, Mittel zur temporären Verformung keratinhaltiger Fasern zur Verfügung zu stellen, welche sich als zielgerichteter Sprühnebel gut auf die keratinhaltigen Fasern applizieren lassen. Weiterhin sollen die Mittel zur temporären Verformung keratinhaltiger Fasern einen hohen Haltegrad, insbesondere Langzeithaltegrad, und einen hohen Volumeneffekt aufweisen.

Es wurde nun überraschend gefunden, dass eine ethanolische Zubereitung einer speziellen Kombination aus hydrophob modifiziertem Metalloxidpulvers mit filmbildendem Polymer zur Lösung der zuvor beschriebenen Aufgabe geeignet ist. Diese spezifische Kombination zeichnet sich nicht nur durch einen hohen Langzeithalt und einem guten Volumeneffekt aus, sondern lässt sich zudem hervorragend vernebeln und eignet sich also auch für die Sprühapplikation. Die Verwendung einer pulverförmigen Zusammensetzung auf Basis hydrophobierten Siliziumdioxidpulvers zur temporären Verformung keratinischer Fasern wird in der internationalen Patentanmeldung WO 2007/051511 A1 beschrieben.

Ein erster Gegenstand der vorliegenden Erfindung ist somit ein kosmetisches Produkt, umfassend ein kosmetisches Mittel zur temporären Verformung keratinischer Fasern, umfassend
a) eine kosmetische Zubereitung, enthaltend, bezogen auf das Gesamtgewicht der kosmetischen Zubereitung,
a1) 0,05 bis 5,0 Gew.-% mindestens eines hydrophob modifizierten Metalloxidpulvers ausgewählt aus den Verbindungen mit den INCI Bezeichnungen Silica Dimethyl Silylate;
a2) 0,1 bis 10 Gew.-% mindestens eines filmbildenden Polymers;
a3) 40 bis 95 Gew.-% Ethanol
a4) weniger als 5 Gew.-% Wasser;
b) eine Abgabevorrichtung mit Sprühventil; und
c) ein Treibmittel.

Die erfindungsgemäßen Mittel enthalten als einen ersten wesentlichen Bestandteil ein hydrophob modifiziertes Metalloxidpulvers ausgewählt aus Verbindungen mit den INCI Bezeichnungen Silica Dimethyl Silylate. Hinsichtlich ihrer kosmetischen Wirkung, Herstellbarkeit und Applizierbarkeit bevorzugte Mittel sind dadurch gekennzeichnet, dass der Gewichtsanteil des hydrophob modifizierten Metalloxidpulvers a1) am Gesamtgewicht der kosmetischen Zubereitung a) 0,1 bis 4,0 Gew.-%, vorzugsweise 0,15 bis 3,0 Gew.-% und insbesondere 0,2 bis 2,0 Gew.-% beträgt.

Unter "hydrophob modifiziert" oder "hydrophobiert" sind im Sinne der Erfindung solche Metalloxide zu verstehen, die zumindest an der Oberfläche der Partikel derart modifiziert wurden, dass das modifizierte Teilchen weniger von Wasser benetzt wird als das nicht modifizierte Teilchen. Insbesondere sind silanisierte, hydrophobierte Metalloxide bevorzugt. Als Reagenz zur Silanisierung des Metalloxids eignet sich erfindungsgemäß bevorzugt mindestens ein Vertreter der Gruppe, die gebildet wird, aus Silanen, Halogensilanen, Alkoxysilanen und Silazanen. Offenbart sind hydrophobierte Metalloxide ausgewählt aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid. Für die Herstellung der erfindungsgemäßen kosmetischen Mittel besonders geeignet haben sich hydrophobierte Silikate erwiesen, wobei durch Silanierung oder durch Umsetzung mit Polydimethylsiloxan nachbehandelte pyrogene Kieselsäure besondere Vorteile aufweisen.

Die erfindungsgemäßen Mittel enthalten hydrophobiertes Siliciumdioxid mit den INCI Bezeichnungen Silica Dimethyl Silylate. Als Reagenz zur Silanisierung des Siliziumdioxids eignen sich bevorzugt mindestens ein Vertreter der Gruppe, die gebildet wird, aus Silanen, Halogensilanen, Alkoxysilanen und Silazanen.

Bevorzugte Vertreter der Gruppe der Silane sind Hexa(C₁-C₂₀)alkyldisilane, insbesondere Hexamethyldisilan.

Wenn ein Halogensilan als Silylierungsmittel Anwendung findet, wird als bevorzugtes Halogensilan mindestens eine Verbindung aus der Gruppe ausgewählt, die gebildet wird, aus den Verbindungen

[(C₁-C₂₀)Alkyl]_{z'}SiX_{(4-z')}

X₃Si[(CH₂)ₙ-R]

X₂[(C₁-C₂₀)Alkyl]Si(CH₂)ₙ-R

[(C₁-C₂₀)Alkyl]_{(y'+1)}[R-(CH₂)ₙ]_{(2-y')}SiX

worin
X ein Chlor-, Brom- oder lodatom bedeutet,
z' eine Zahl 1, 2 oder 3 ist,
y' eine Zahl 0, 1 oder 2 ist
n eine ganze Zahl von 1 bis 20 ist und
R steht für einen Rest aus
(C₁-C₁₀)Alkyl-, Aryl-, (C₁-C₆)Perfluoroalkyl-, -NH₂, -N₃, -SCN, -CH=CH₂, -O(O)C-C(CH₃)=CH₂, -OCH₂-CH=CH₂, 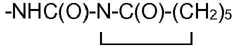
-NH-C(O)O-Me, -NH-C(O)O-Et, -NH-(CH₂)₃-Si(O(C₁-C₆)alkyl)₃.

Wenn ein Alkoxysilan als Silylierungsmittel verwendet wird, wird als bevorzugtes Alkoxysilan mindestens eine Verbindung aus der Gruppe ausgewählt, die gebildet wird, aus den Verbindungen

[(C₁-C₂₀)AlkylO]_{z}Si(C₁-C₂₀)Alkyl_{(4-z)}

[(C₁-C₂₀)AlkylO]_{z}Si[(CH₂)ₙ-R]_{(4-z)}

[(C₁-C₂₀)AlkylO]₂[(C₁-C₂₀)Alkyl]Si(CH₂)ₙ-R

[(C₁-C₂₀)AlkylO][(C₁-C₂₀)Alkyl]₂Si(CH₂)ₙ-R

[(C₁-C₂₀)AlkylO][(C₁-C₂₀)Alkyl]Si[(CH₂)ₙ-R]₂

(C₁-C₂₀Alkyl)₃SiO-C(CH₃)=N-Si(C₁-C₂₀)Alkyl₃,

worin
n eine ganze Zahl von 1 bis 20 ist und
z eine Zahl 1, 2, oder 3 bedeutet
R steht für einen Rest aus
(C₁-C₂₀)Alkyl-, Aryl-, (C₁-C₆)Perfluoroalkyl-, -NH₂, -N₃, -SCN, -CH=CH₂, -O(O)C-C(CH₃)=CH₂, -OCH₂-CH=CH₂, 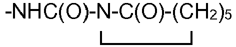
-NH-C(O)O-Me, -NH-C(O)O-Et, -NH-(CH₂)₃-Si(O(C₁-C₆)alkyl)₃.

Als bevorzugtes Silazan wird mindestens eine Verbindung aus der Klasse der Disilazane, insbesondere mindestens eine Verbindung aus Disilazanen der Formel

R'₂R"Si-NH-SiR'₂R"

ausgewählt, worin
R' eine (C₁-C₂₀)Alkylgruppe bedeutet und
R" eine (C₁-C₂₀)Alkylgruppe oder eine Vinylgruppe bedeutet. Ein besonders bevorzugtes Silazan ist Hexamethyldisilazan.

Alle oben genannten Alkylgruppen, ob (C₁-C₆)Alkyl, (C₁-C₁₀)Alkyl oder (C₁-C₂₀)-Alkyl, können sowohl zyklisch, als auch linear bzw. verzweigt sein. Beispiele für verwendbare Alkylgruppen sind Methyl, Ethyl, n-Propyl, Isopropyl, n-Butyl, Cyclopentyl, Cyclohexyl, n-Decyl, Lauryl, Myristyl, Cetyl, Stearyl, Isostearyl und Behenyl.

Ein Beispiel für eine Arylgruppe ist die Phenylgruppe. Beispiele für eine (C₁-C₆)Perfluoroalkylgruppe sind Trifluormethyl, Perfluoroethyl, Perfluoropropyl und Perfluorohexyl.

Vorzugsweise werden hydrophobierte Siliziumdioxide eingesetzt, die durch Silanisierung von pyrogenem Siliziumdioxid erhalten werden.

Offenbart sind silanisierte, hydrophobierte Siliziumdioxide ausgewählt aus mindestens einer Verbindung der Gruppe, die gebildet wird aus Trimethylsilylat-beschichtetem Siliziumdioxid, Dimethylsilylat-beschichtetem Siliziumdioxid, Octylsilylat-beschichtetem Siliziumdioxid.

Eine Vielzahl hydrophob modifizierter Siliciumdioxide ist kommerziell erhältlich. Beispielhaft seien Aerosil® R104 V, Aerosil® R106, Aerosil® R202, Aerosil® R805, Aerosil® R812, Aerosil® R812S, Aerosil® R972 und Aerosil® R8200, alle Degussa, sowie HDK® H2000, HDK® H2050 und HDK® H3004, alle Wacker, genannt. Erfindungsgemäß kommt das Siliciumdioxid mit der INCI-Bezeichnung Silica Dimethyl Silylate zum Einsatz, das von der Firma Degussa unter der Bezeichnung Aerosil® R972 vertrieben wird.

Die hydrophob modifizierten Siliciumdioxide mit der INCI Bezeichnung Silica Dimethyl Silylate ermöglich eine, im Vergleich zu den übrigen zuvor beschriebenen hydrophob modifizierten Siliciumdioxiden, vereinfachte Herstellung erfindungsgemäßer kosmetischer Mittel, welche sich zudem durch eine verbesserte Applizierbarkeit und kosmetische Wirkung auszeichnen. Die Herstellung der besonders bevorzugten hydrophobierten Siliciumdioxide mit der INCI Bezeichnung Silica Dimethyl Silylate kann beispielsweise durch Umsetzung pyrogener Kieselsäure mit Dimethyldichlorsilan erfolgen. Ein besonders bevorzugter Gegenstand der vorliegenden Anmeldung ist daher ein kosmetisches Mittel, welches ein hydrophob modifiziertes Metalloxidpulver enthält, das durch Umsetzung pyrogener Kieselsäure mit Dimethyldichlorsilan erhalten wird.

Der Partikeldurchmesser der Primärteilchen bevorzugter hydrophob modifizierter Metalloxide a1), insbesondere der hydrophob modifizierten Metalloxide mit der INCI Bezeichnung Silica Dimethyl Silylate beträgt vorzugsweise weniger als 5 µm, besonders bevorzugt weniger als 1 µm, und insbesondere zwischen 1 und 50 nm.

Bevorzugt sind weiterhin solche hydrophob modifizierten Metalloxide a1), insbesondere hydrophob modifizierte Metalloxide mit der INCI Bezeichnung Silica Dimethyl Silylate, die eine spezifische Oberfläche nach BET zwischen 10 und 400 m²/g, vorzugsweise zwischen 40 bis 300 m²/g und insbesondere 80 bis 150 m²/g aufweisen.

Die erfindungsgemäßen pulverförmigen Zusammensetzungen enthalten als zweiten wesentlichen Bestandteil ein filmbildendes Polymer a2). Für die Anwendung und die kosmetische Wirkung der kosmetischen Mittel hat es sich als vorteilhaft erwiesen, wenn der Gewichtsanteil des filmbildenden Polymers a2) am Gesamtgewicht der kosmetischen Zubereitung a) 0,2 bis 8,0 Gew.-%, vorzugsweise 0,4 bis 6,0 Gew.-% und insbesondere 0,5 bis 5,0 Gew.-% beträgt.

Ein aufgrund seiner kosmetischen Wirkung besonders bevorzugtes filmbildendes Polymer a2) ist ein durch Polymerisation der Monomere N-tert-Octylacrylamid, Acrylsäure und tert.-Butylaminoethylmethacrylsäure erhaltenes Copolymer. Bevorzugte filmbildende Poylmere a2) bestehen zu mindestens 90 Gew.-%, vorzugsweise zu mindestens 95 Gew.-% und insbesondere zu mindestens 97 Gew.-% aus den Monomeren N-tert-Octylacrylamid, Acrylsäure und tert.-Butylaminoethylmethacrylsäure. Besonders bevorzugte filmbildende Polymere a2) wurden ausschließlich aus den Monomeren N-tert-Octylacrylamid, Acrylsäure und tert.-Butylaminoethylmethacrylsäure erhalten. Die zuvor beschriebenen besonders bevorzugten filmbildenden Polymere a2) werden beispielsweise unter der Bezeichnung Amphomer® (INCI-Bezeichnung: Octylacrylamide / Acrylates / Butylaminoethyl Methacrylate Copolymer) von der Firma National Starch vertrieben.

Bevorzugte kosmetische Mittel sind dadurch gekennzeichnet, dass es sich bei dem filmbildenden Polymer a2) um ein Copolymer aus den Monomeren
i) N-tert-Octylacrylamid
ii) Acrylsäure
iii) tert.-Butylaminoethylmethacrylsäure
iv) sowie gegebenenfalls weiteren Monomeren
handelt.

Bevorzugte kosmetische Mittel enthalten als filmbildendes Polymer a2) zu mindestens 50 Gew.-%, vorzugsweise zu mindestens 70 Gew.-%, besonders bevorzugt zu mindestens 90 Gew.% und insbesondere zu mindestens 95 Gew.-% ein durch Polymerisation der Monomere N-tert-Octylacrylamid, Acrylsäure und tert.-Butylaminoethylmethacrylsäure erhaltenes Copolymer. Besonders bevorzugt werden kosmetische Mittel, welche als filmbildendes Polymer a2) ausschließlich ein durch Polymerisation der Monomere N-tert-Octylacrylamid, Acrylsäure und tert.-Butylaminoethylmethacrylsäure erhaltenes Copolymer enthalten.

Als weitere brauchbare filmbildende Polymere a2), die alternativ zu den Copolymeren von N-tert-Octylacrylamid, Acrylsäure und tert.-Butylaminoethylmethacrylsäure oder in Kombination mit diesen Copolymeren eingesetzt werden können haben sich
- Copolymer aus der Gruppe der Polyvinylpyrrolidon und Vinylpyrrolidon/VinylacetatCopolymere, vorzugsweise der Polyvinylpyrrolidone
- Copolymer aus den Monomeren Acrylsäure, Ethylacrylat und tert-Butylacrylamid erwiesen.

Weitere bevorzugte kosmetische Mittel sind daher dadurch gekennzeichnet, dass es sich bei dem filmbildenden Polymer a2)
- um ein Copolymer aus der Gruppe der Polyvinylpyrrolidon und Vinylpyrrolidon/VinylacetatCopolymere, vorzugsweise der Polyvinylpyrrolidone bzw.
- um ein Copolymer aus den Monomeren
   i) Acrylsäure
   ii) Ethylacrylat
   iii) tert-Butylacrylamid handelt.

Die filmbildenden Polymere a2) werden in den kosmetischen Mitteln vorzugsweise in teilneutralisierter oder neutralisierter Form eingesetzt. Zur Neutralisation wird bevorzugt mindestens ein Alkanolamin verwendet. Die als Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugte kosmetische Mittel enthalten daher 2-Amino-2-methylpropanol. Das 2-Amino-2-methylpropanol wird in den erfindungsgemäßen Mitteln vorzugsweise in einer Menge eingesetzt, welche die zur Neutralisation der filmbildenden Polymere a2) benötigte Menge nicht überschreitet. Vorzugsweise beträgt die in den erfindungsgemäßen Mitteln eingesetzte Mengen an 2-Amino-2-methylpropanol 80 bis 100%, besonders bevorzugt 90 bis 100% und insbesondere 95 bis 100% der zur vollständigen Neutralisation der filmbildenden Polymere a2) benötigten Menge. In einer bevorzugten Ausführungsform beträgt der Gewichtsanteil des 2-Amino-2-methylpropanols am Gesamtgewicht der kosmetischen Zubereitung 0,1 bis 4,0 Gew.-%, bevorzugt 0,2 bis 3,0 Gew.% und insbesondere 0,4 bis 2,0 Gew.-%.

Als dritten wesentlichen Bestandteil enthalten erfindungsgemäße kosmetische Mittel 40 bis 95 Gew.-% Ethanol, wobei es in Bezug auf die Herstellung und Applikation der kosmetischen Mittel bevorzugt ist, dass der Gewichtsanteil des Ethanols a3) am Gesamtgewicht der kosmetischen Zubereitung a) 50 bis 95 Gew.-%, vorzugsweise 70 bis 95 Gew.-% und insbesondere 80 bis 95 Gew.-% beträgt. Der Gewichtsanteil von Wasser am Gesamtgewicht der kosmetischen Zubereitung a) beträgt dem gegenüber weniger als 5 Gew.-%.

Erfindungsgemäße Mittel liegen in Form fließfähiger Suspensionen vor.

Neben den zuvor beschriebenen Inhaltsstoffen können die erfindungsgemäßen kosmetischen Mittel weitere Wirk-, Hilfs- und Pflegestoffe enthalten.

Eine erste Gruppe bevorzugter weiterer Wirkstoffe bilden die Poly-(C₂-C₃)alkylenglycol-modifizierten Silicone a4). Besonders bevorzugt sind Poly-(C₂-C₃)alkylenglycol-modifizierten Silicone aus der Gruppe der alkoxylierten Dimethicone, insbesondere der
- ethoxylierten Dimethicone mit der INCI-Bezeichnung PEG-x Dimethicone mit x = 2 bis 20, vorzugsweise 3 bis 17 und insbesondere 11 oder 12;
- der ethoxylierten Dimethicone mit der INCI-Bezeichnung Bis-PEG-y Dimethicone mit x= 3 bis 25, vorzugsweise 4 bis 20;
- der ethoxylierten/propoxylierten Dimethicone mit der INCI-Bezeichnung PEG/PPG a/b Dimethicone, wobei a und b unabhängig voneinander für Zahlen von 2 bis 30, vorzugsweise von 12 bis 24 und insbesondere von 14 bis 20 stehen;
- der ethoxylierten/propoxylierten Dimethicone mit der INCI-Bezeichnung Bis-PEG/PPG-c/d Dimethicone, wobei c und d unabhängig voneinander für Zahlen von 10 bis 25, bevorzugt 14 bis 20 und insbesondere von 14 bis 16 stehen
- der ethoxylierten/propoxylierten Dimethicone mit der INCI-Bezeichnung Bis-PEG/PPG-e/f PEG/PPG g/h Dimethicone, wobei e, f, g und h unabhängig voneinander für Zahlen von 10 bis 20, vorzugsweise 14 bis 18 und insbesondere 16 stehen.

Besonders bevorzugt ist der Einsatz von ethoxylierten Dimethiconen mit der INCI-Bezeichnung PEG-12 Dimethicone. Der Gewichtsanteil der Poly-(C₂-C₃)alkylenglycol-modifizierten Silicone a4), vorzugsweise der ethoxylierten Dimethicone, insbesondere des PEG-12 Dimethicons am Gesamtgewicht der kosmetischen Zubereitung a) beträgt vorzugsweise 0,0001 bis 1,0 Gew.-%, bevorzugt 0,0002 bis 0,8 Gew.-% und insbesondere 0,0005 bis 0,5 Gew.-%.

Eine weitere Gruppe bevorzugter Wirkstoffe bilden die Esteröle a5). Unter Esterölen sind dabei die Ester von C₆ - C₃₀ - Fettsäuren mit C₂ - C₃₀ - Fettalkoholen zu verstehen. Bevorzugt sind die Monoester der Fettsäuren mit Alkoholen mit 2 bis 24 C-Atomen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V). In bevorzugten erfindungsgemäßen kosmetischen Mitteln beträgt der Gewichtsanteil des Esteröls a5) am Gesamtgewicht der kosmetischen Zubereitung a) 0,01 bis 1,0 Gew.-%, vorzugsweise 0,02 bis 0,8 Gew.-% und insbesondere 0,04 bis 0,4 Gew.-%.

Als Pflegestoff können Proteinhydrolysate und/oder deren Derivate eingesetzt werden. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Ein weitere Gruppe von Pflegestoffen sind die Vitamine, Provitamine, Vitaminvorstufen und/oder deren Derivate. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Glycerin, Propylenglykol, Panthenol, Coffein, Nicotinamid und Sorbitol.

Als Pflegestoff können Pflanzenextrakt, aber auch Mono- bzw. Oligosaccharide und/oder Lipide eingesetzt werden.

Die erfindungsgemäßen kosmetischen Mittel eignen sich in besonderer Weise zur Applikation mittels einer Sprühvorrichtung, wobei es sich bei der eingesetzten Sprühvorrichtung um ein Pumpspray oder ein Aerosolspray handeln kann. Entsprechende kosmetische Produkte umfassen demnach neben dem zuvor beschriebenen kosmetischen Mittel a) weiterhin eine Abgabevorrichtung mit Sprühventil b).

Bevorzugt ist der Einsatz von Aerosolsprays, bei denen das kosmetische Mittel a) mittels eines unter Druck stehenden Treibgases versprüht wird. Entsprechende kosmetische Produkte umfassen neben dem zuvor beschriebenen kosmetisches Mittel a) und einer Abgabevorrichtung mit Sprühventil b) weiterhin ein Treibmittel c).

Erfindungsgemäß geeignete Treibmittel (Treibgase) sind Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, Mischungen dieser Treibgase sowie Dimethylether. Es hat sich gezeigt, dass der Einsatz von Dimethylether als einzigem Treibgas Vorteile in der Herstellung und der Anwendung mit sich bringt.

Vorzugsweise beträgt das Gewichtsverhältnis der kosmetischen Zubereitung a) zum Treibmittel c) 5:1 bis 1:3, vorzugsweise 4:1 bis 1:2 und insbesondere 2:1 bis 1:1. Bevorzugte kosmetische Mittel umfassen demnach
- eine kosmetische Zubereitung a), enthaltend, bezogen auf das Gesamtgewicht der kosmetischen Zubereitung,
   a1) 0,05 bis 5,0 Gew.-% mindestens eines hydrophob modifiziertes Metalloxidpulver;
   a2) 0,1 bis 10 Gew.-% mindestens eines filmbildenden Polymers;
   a3) 40 bis 95 Gew.-% Ethanol.
- ein Treibmittel c), vorzugsweise Dimethylether,
wobei das Gewichtsverhältnis der kosmetischen Zubereitung a) zum Treibmittel c) 5:1 bis 1:3, vorzugsweise 4:1 bis 1:2 und insbesondere 2:1 bis 1:1 beträgt.

Im Falle der Aerosolsprays umfasst die Abgabevorrichtung mit Sprühventil regelmäßig auch einen Druckgasbehälter. Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber dem kosmetischen Mittel a).

In einer bevorzugten Ausführungsform der Erfindung weist das Ventil einen mit einem Lack oder einem polymeren Kunststoff A beschichteten Ventilkegel und ein ebensolches flexibles Element mit Rückstellcharakteristik auf, dass das Ventil nach Beenden der Betätigung in die Verschlussstellung (= Ruhelage des Ventils) zurückstellt. Entsprechende kosmetische Produkte, bei denen die Aerosol-Abgabevorrichtung ein Ventil umfasst, das einen Ventilkegel und/oder ein flexibles Element mit Rückstellcharakteristik aufweist, der/das/die mit einem Lack oder einem polymeren Kunststoff A beschichtet ist/sind, sind erfindungsgemäß bevorzugt.

In einer weiteren bevorzugten Ausführungsform der Erfindung weist das Ventil ein flexibles Element mit Rückstellcharakteristik und/oder einen Ventilkegel aus mindestens einem Kunststoff B, bevorzugt einem elastomeren Kunststoff, auf. Auch hier sind erfindungsgemäße kosmetische Produkte, bei denen das Ventil ein flexibles Element mit Rückstellcharakteristik und/oder einen Ventilkegel aus mindestens einem Kunststoff B aufweist, bevorzugt, wobei bevorzugte Kunststoffe B elastomere Kunststoffe sind. Besonders bevorzugte elastomere Kunststoffe sind ausgewählt aus Buna, insbesondere Buna N, Buna 421, Buna 1602 und Buna KA 6712, Neopren, Butyl und Chlorbutyl.

In einer weiteren bevorzugten Ausführungsform der Erfindung kann das flexible Element mit Rückstellcharakteristik als Spiralfeder bzw. Schraubendruckfeder ausgebildet sein. In einer weiteren bevorzugten Ausführungsform der Erfindung kann das flexible Element mit Rückstellcharakteristik einstückig mit dem Ventilkegel ausgebildet sein und biegsame Beine aufweisen.

Die Zusammensetzung einiger bevorzugter kosmetischer Zubereitungen kann den folgenden Tabellen entnommen werden (Angaben in Gew.-% bezogen auf das Gesamtgewicht der kosmetischen Zubereitung sofern nicht anders angegeben). Die Beispiele 1-15 und 26-40 sind Referenzbeispiele und dienen der Illustration.

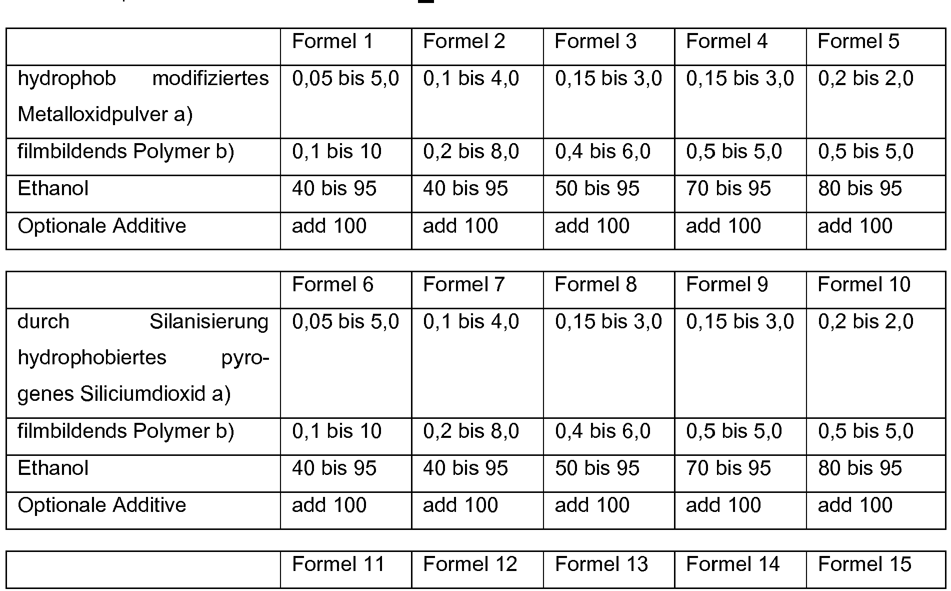

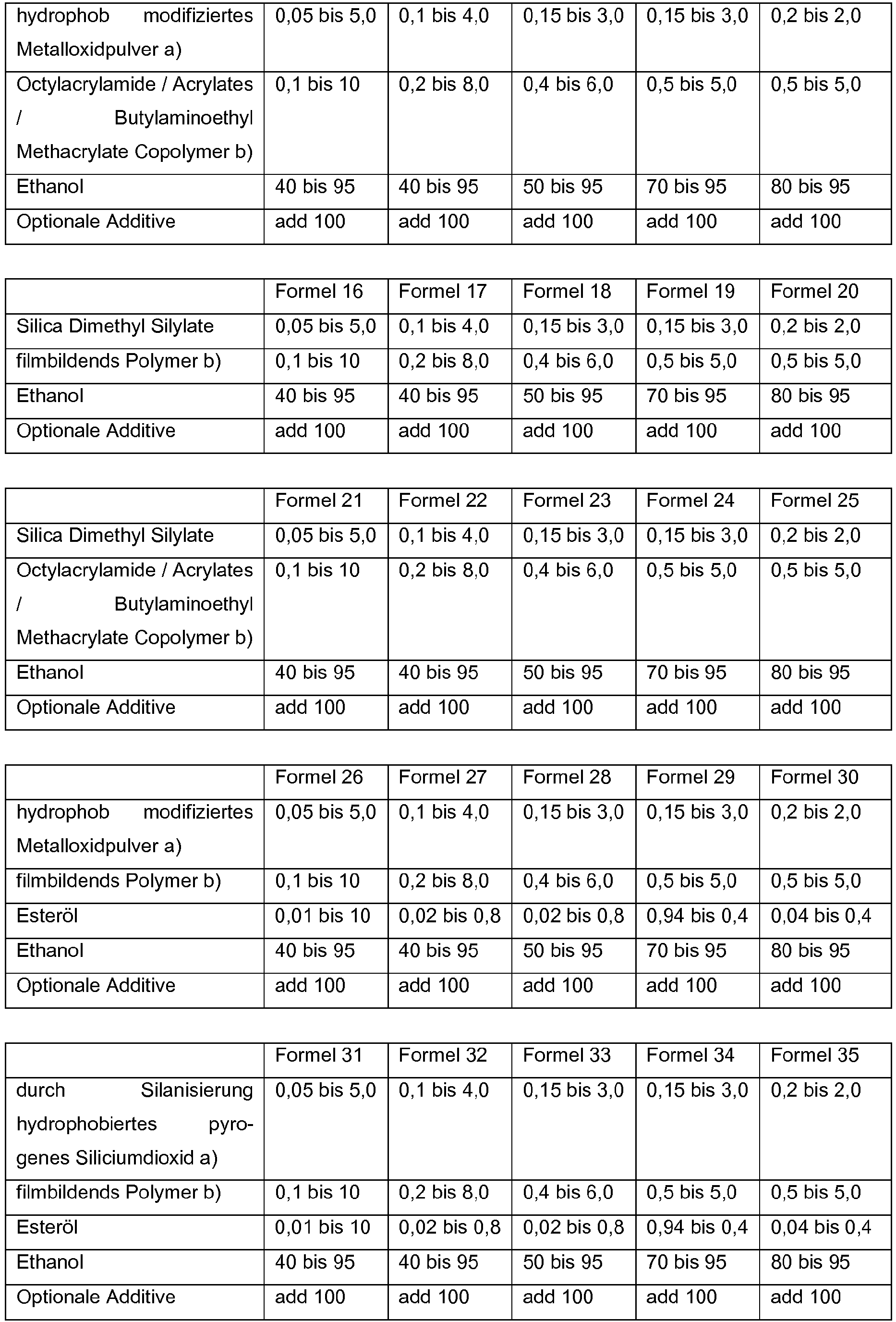

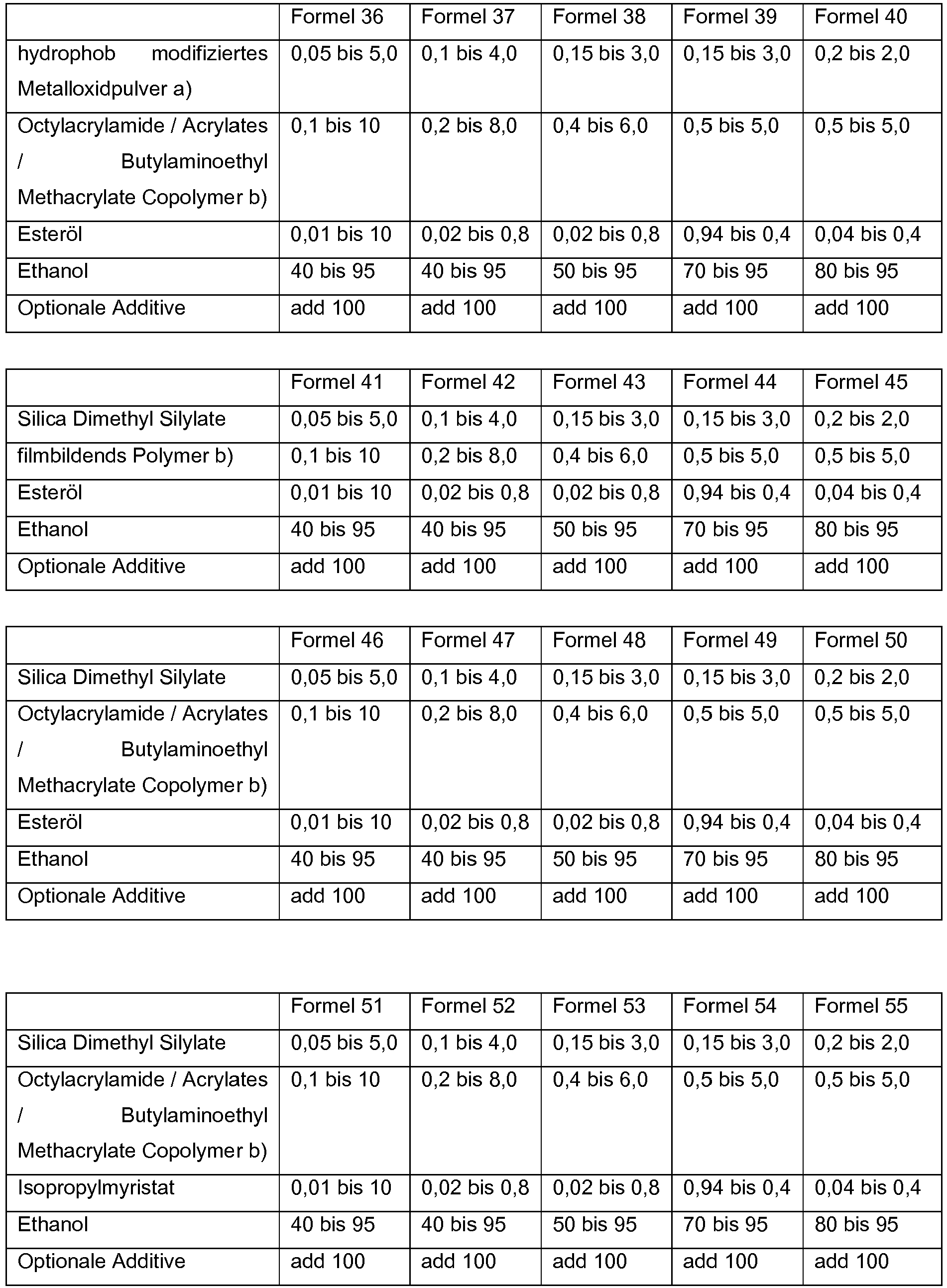

Die weitere Zusammensetzung einiger bevorzugter kosmetischer Mittel, welche neben der kosmetischen Zubereitung a) weiterhin ein Treibmittel c) umfassen, kann der folgenden Tabellen entnommen werden. In dieser Tabelle verweist die linke Spalte ("Formel x") auf jeweils eine der in den weiter oben offenbarten Tabellen angeführten beispielhaften kosmetischen Zubereitungen. Die weiteren Spalten zwei bis sieben ("Treibmittel") geben jeweils die der entsprechenden kosmetischen Zubereitung zugesetzte Menge an Treibmittel an. Diese Angaben in "Gew.-%" beziehen sich auf das Gesamtgewicht der kosmetischen Zubereitung a) der jeweiligen "Formel x" ohne Treibmittel.

Bei den kosmetischen Mitteln gemäß Zeile 2, Spalte 6 der nachfolgenden Tabelle handelt es sich mit anderen Worten um Mischungen der Treibmittel-freien kosmetischen Zubereitung a) gemäß Formel 1 mit Dimethylether in einem Gewichtsverhältnis von 2:1 bis 1:1.

In Zeile 4, Spalte 5 der nachfolgenden Tabelle ist dem gegenüber eine 3:2 Mischung der Treibmittel-freien kosmetischen Zubereitung a) gemäß Formel 3 mit einem Propan/Butan Gemisch beschrieben.

| | Treibmittel [Gew.-%] | | | | | |
|---|---|---|---|---|---|---|
| Formel 1 | 50 bis 100 | 66,6 | 50 bis 100 P/B* | 66,6 P/B | 50 bis 100 DME** | 66,6 DME |
| Formel 2 | 50 bis 100 | 66,6 | 50 bis 100 P/B* | 66,6 P/B | 50 bis 100 DME** | 66,6 DME |
| Formel 3 | 50 bis 100 | 66,6 | 50 bis 100 P/B* | 66,6 P/B | 50 bis 100 DME** | 66,6 DME |
| Formel 4 | 50 bis 100 | 66,6 | 50 bis 100 P/B* | 66,6 P/B | 50 bis 100 DME** | 66,6 DME |
| Formel 5 | 50 bis 100 | 66,6 | 50 bis 100 P/B* | 66,6 P/B | 50 bis 100 DME** | 66,6 DME |
| Formel 6 | 50 bis 100 | 66,6 | 50 bis 100 P/B* | 66,6 P/B | 50 bis 100 DME** | 66,6 DME |
| Formel 7 | 50 bis 100 | 66,6 | 50 bis 100 P/B* | 66,6 P/B | 50 bis 100 DME** | 66,6 DME |
| Formel 8 | 50 bis 100 | 66,6 | 50 bis 100 P/B* | 66,6 P/B | 50 bis 100 DME** | 66,6 DME |
| Formel 9 | 50 bis 100 | 66,6 | 50 bis 100 P/B* | 66,6 P/B | 50 bis 100 DME** | 66,6 DME |
| Formel 10 | 50 bis 100 | 66,6 | 50 bis 100 P/B* | 66,6 P/B | 50 bis 100 DME** | 66,6 DME |
| Formel 11 | 50 bis 100 | 66,6 | 50 bis 100 P/B* | 66,6 P/B | 50 bis 100 DME** | 66,6 DME |
| Formel 12 | 50 bis 100 | 66,6 | 50 bis 100 P/B* | 66,6 P/B | 50 bis 100 DME** | 66,6 DME |
| Formel 13 | 50 bis 100 | 66,6 | 50 bis 100 P/B* | 66,6 P/B | 50 bis 100 DME** | 66,6 DME |
| Formel 14 | 50 bis 100 | 66,6 | 50 bis 100 P/B* | 66,6 P/B | 50 bis 100 DME** | 66,6 DME |
| Formel 15 | 50 bis 100 | 66,6 | 50 bis 100 P/B* | 66,6 P/B | 50 bis 100 DME** | 66,6 DME |
| Formel 16 | 50 bis 100 | 66,6 | 50 bis 100 P/B* | 66,6 P/B | 50 bis 100 DME** | 66,6 DME |
| Formel 17 | 50 bis 100 | 66,6 | 50 bis 100 P/B* | 66,6 P/B | 50 bis 100 DME** | 66,6 DME |
| Formel 18 | 50 bis 100 | 66,6 | 50 bis 100 P/B* | 66,6 P/B | 50 bis 100 DME** | 66,6 DME |
| Formel 19 | 50 bis 100 | 66,6 | 50 bis 100 P/B* | 66,6 P/B | 50 bis 100 DME** | 66,6 DME |
| Formel 20 | 50 bis 100 | 66,6 | 50 bis 100 P/B* | 66,6 P/B | 50 bis 100 DME** | 66,6 DME |
| Formel 21 | 50 bis 100 | 66,6 | 50 bis 100 P/B* | 66,6 P/B | 50 bis 100 DME** | 66,6 DME |
| Formel 22 | 50 bis 100 | 66,6 | 50 bis 100 P/B* | 66,6 P/B | 50 bis 100 DME** | 66,6 DME |
| Formel 23 | 50 bis 100 | 66,6 | 50 bis 100 P/B* | 66,6 P/B | 50 bis 100 DME** | 66,6 DME |
| Formel 24 | 50 bis 100 | 66,6 | 50 bis 100 P/B* | 66,6 P/B | 50 bis 100 DME** | 66,6 DME |
| Formel 25 | 50 bis 100 | 66,6 | 50 bis 100 P/B* | 66,6 P/B | 50 bis 100 DME** | 66,6 DME |
| Formel 26 | 50 bis 100 | 66,6 | 50 bis 100 P/B* | 66,6 P/B | 50 bis 100 DME** | 66,6 DME |
| Formel 27 | 50 bis 100 | 66,6 | 50 bis 100 P/B* | 66,6 P/B | 50 bis 100 DME** | 66,6 DME |
| Formel 28 | 50 bis 100 | 66,6 | 50 bis 100 P/B* | 66,6 P/B | 50 bis 100 DME** | 66,6 DME |
| Formel 29 | 50 bis 100 | 66,6 | 50 bis 100 P/B* | 66,6 P/B | 50 bis 100 DME** | 66,6 DME |
| Formel 30 | 50 bis 100 | 66,6 | 50 bis 100 P/B* | 66,6 P/B | 50 bis 100 DME** | 66,6 DME |
| Formel 31 | 50 bis 100 | 66,6 | 50 bis 100 P/B* | 66,6 P/B | 50 bis 100 DME** | 66,6 DME |
| Formel 32 | 50 bis 100 | 66,6 | 50 bis 100 P/B* | 66,6 P/B | 50 bis 100 DME** | 66,6 DME |
| Formel 33 | 50 bis 100 | 66,6 | 50 bis 100 P/B* | 66,6 P/B | 50 bis 100 DME** | 66,6 DME |
| Formel 34 | 50 bis 100 | 66,6 | 50 bis 100 P/B* | 66,6 P/B | 50 bis 100 DME** | 66,6 DME |
| Formel 35 | 50 bis 100 | 66,6 | 50 bis 100 P/B* | 66,6 P/B | 50 bis 100 DME** | 66,6 DME |
| Formel 36 | 50 bis 100 | 66,6 | 50 bis 100 P/B* | 66,6 P/B | 50 bis 100 DME** | 66,6 DME |
| Formel 37 | 50 bis 100 | 66,6 | 50 bis 100 P/B* | 66,6 P/B | 50 bis 100 DME** | 66,6 DME |
| Formel 38 | 50 bis 100 | 66,6 | 50 bis 100 P/B* | 66,6 P/B | 50 bis 100 DME** | 66,6 DME |
| Formel 39 | 50 bis 100 | 66,6 | 50 bis 100 P/B* | 66,6 P/B | 50 bis 100 DME** | 66,6 DME |
| Formel 40 | 50 bis 100 | 66,6 | 50 bis 100 P/B* | 66,6 P/B | 50 bis 100 DME** | 66,6 DME |
| Formel 41 | 50 bis 100 | 66,6 | 50 bis 100 P/B* | 66,6 P/B | 50 bis 100 DME** | 66,6 DME |
| Formel 42 | 50 bis 100 | 66,6 | 50 bis 100 P/B* | 66,6 P/B | 50 bis 100 DME** | 66,6 DME |
| Formel 43 | 50 bis 100 | 66,6 | 50 bis 100 P/B* | 66,6 P/B | 50 bis 100 DME** | 66,6 DME |
| Formel 44 | 50 bis 100 | 66,6 | 50 bis 100 P/B* | 66,6 P/B | 50 bis 100 DME** | 66,6 DME |
| Formel 45 | 50 bis 100 | 66,6 | 50 bis 100 P/B* | 66,6 P/B | 50 bis 100 DME** | 66,6 DME |
| Formel 46 | 50 bis 100 | 66,6 | 50 bis 100 P/B* | 66,6 P/B | 50 bis 100 DME** | 66,6 DME |
| Formel 47 | 50 bis 100 | 66,6 | 50 bis 100 P/B* | 66,6 P/B | 50 bis 100 DME** | 66,6 DME |
| Formel 48 | 50 bis 100 | 66,6 | 50 bis 100 P/B* | 66,6 P/B | 50 bis 100 DME** | 66,6 DME |
| Formel 49 | 50 bis 100 | 66,6 | 50 bis 100 P/B* | 66,6 P/B | 50 bis 100 DME** | 66,6 DME |
| Formel 50 | 50 bis 100 | 66,6 | 50 bis 100 P/B* | 66,6 P/B | 50 bis 100 DME** | 66,6 DME |
| Formel 51 | 50 bis 100 | 66,6 | 50 bis 100 P/B* | 66,6 P/B | 50 bis 100 DME** | 66,6 DME |
| Formel 52 | 50 bis 100 | 66,6 | 50 bis 100 P/B* | 66,6 P/B | 50 bis 100 DME** | 66,6 DME |
| Formel 53 | 50 bis 100 | 66,6 | 50 bis 100 P/B* | 66,6 P/B | 50 bis 100 DME** | 66,6 DME |
| Formel 54 | 50 bis 100 | 66,6 | 50 bis 100 P/B* | 66,6 P/B | 50 bis 100 DME** | 66,6 DME |
| Formel 55 | 50 bis 100 | 66,6 | 50 bis 100 P/B* | 66,6 P/B | 50 bis 100 DME** | 66,6 DME |

| | | | | | | |
|---|---|---|---|---|---|---|
| * "P/B" entspricht einem Propan/Butan Gemisch ** "DME" entspricht Dimethylether | | | | | | |

Wie eingangs ausgeführt zeichnen sich die zuvor beschriebenen kosmetischen Mittel durch besondere haarkosmetische Eigenschaften, insbesondere vorteilhafte Eigenschaften bei der temporären Haarverformung aus. Ein zweiter Gegenstand der vorliegenden Anmeldung ist daher die Verwendung eines erfindungsgemäßen Mittels zur temporären Verformung keratinhaltiger Fasern, insbesondere menschlicher Haare.

Ein dritter Gegenstand der vorliegenden Anmeldung ist ein Verfahren zur temporären Verformung keratinhaltiger Fasern, insbesondere menschlicher Haare, bei welchem die keratinischen Fasern mit einem erfindungsgemäßen kosmetischen Mittel beaufschlagt und temporär in ihrer Form fixiert werden.

## Patentansprüche

1. Kosmetisches Produkt, umfassend ein kosmetisches Mittel zur temporären Verformung keratinischer Fasern, umfassend
a) eine kosmetische Zubereitung, enthaltend, bezogen auf das Gesamtgewicht der kosmetischen Zubereitung,
a1) 0,05 bis 5,0 Gew.-% mindestens eines hydrophob modifizierten Metalloxidpulvers ausgewählt aus den Verbindungen mit den INCI Bezeichnungen Silica Dimethyl Silylate;
a2) 0,1 bis 10 Gew.-% mindestens eines filmbildenden Polymers;
a3) 40 bis 95 Gew.-% Ethanol
a4) weniger als 5 Gew.-% Wasser;
b) eine Abgabevorrichtung mit Sprühventil; und
c) ein Treibmittel.

2. Kosmetisches Mittel nach Anspruch 1, **dadurch gekennzeichnet, dass** der Gewichtsanteil des hydrophob modifizierten Metalloxidpulvers a1) am Gesamtgewicht der kosmetischen Zubereitung a) 0,1 bis 4,0 Gew.-%, vorzugsweise 0,15 bis 3,0 Gew.-% und insbesondere 0,2 bis 2,0 Gew.-% beträgt.

3. Kosmetisches Mittel nach einem der vorherigen Ansprüche, **dadurch gekennzeichnet, dass** der Gewichtsanteil des filmbildenden Polymers a2) am Gesamtgewicht der kosmetischen Zubereitung a) 0,2 bis 8,0 Gew.-%, vorzugsweise 0,4 bis 6,0 Gew.-% und insbesondere 0,5 bis 5,0 Gew.-% beträgt.

4. Kosmetisches Mittel nach einem der vorherigen Ansprüche, **dadurch gekennzeichnet, dass** es sich bei dem filmbildenden Polymer a2) um ein Copolymer aus den Monomeren
i) N-tert-Octylacrylamid
ii) Acrylsäure
iii) tert.-Butylaminoethylmethacrylsäure
iv) sowie gegebenenfalls weiteren Monomeren
handelt.

5. Kosmetisches Mittel nach einem der vorherigen Ansprüche, **dadurch gekennzeichnet, dass** der Gewichtsanteil des Ethanols a3) am Gesamtgewicht der kosmetischen Zubereitung a) 50 bis 95 Gew.-%, vorzugsweise 70 bis 95 Gew.-% und insbesondere 80 bis 95 Gew.-% beträgt.

6. Verwendung eines Produkts nach einem der Ansprüche 1 bis 5 zur temporären Verformung keratinhaltiger Fasern, insbesondere menschlicher Haare.

7. Verfahren zur temporären Verformung keratinhaltiger Fasern, insbesondere menschlicher Haare, bei welchem die keratinischen Fasern mit einem kosmetischen Mittel nach einem der Ansprüche 1 bis 5 beaufschlagt und temporär in ihrer Form fixiert werden, indem das kosmetische Mittel mittels eines unter Druck stehenden Treibmittels versprüht wird.

## Claims

1. A cosmetic product, comprising a cosmetic agent for temporarily shaping keratin fibers, comprising
a) a cosmetic preparation containing, based on the total weight of the cosmetic preparation,
a1) 0.05 to 5.0 wt.% of at least one hydrophobically modified metal oxide powder, selected from the compounds having the INCI names silica dimethyl silylate;
a2) 0.1 to 10 wt.% of at least one film-forming polymer;
a3) 40 to 95 wt.% ethanol;
a4) less than 5 wt.% water;
b) a dispensing device having a spray valve; and
c) a propellant.

2. The cosmetic agent according to claim 1, **characterized in that** the weight percentage of the hydrophobically modified metal oxide powder a1) in the total weight of the cosmetic preparation a) is 0.1 to 4.0 wt.%, preferably 0.15 to 3.0 wt.% and in particular 0.2 to 2.0 wt.%.

3. The cosmetic agent according to either of the preceding claims, **characterized in that** the weight percentage of the film-forming polymer a2) in the total weight of the cosmetic preparation a) is 0.2 to 8.0 wt.%, preferably 0.4 to 6.0 wt.% and in particular 0.5 to 5.0 wt.%.

4. The cosmetic agent according to one of the preceding claims, **characterized in that** the film-forming polymer a2) is a copolymer from the monomers
i) N-tert-octylacrylamide
ii) acrylic acid
iii) tert-butylaminoethyl methacrylic acid
iv) and optionally further monomers.

5. The cosmetic agent according to one of the preceding claims, **characterized in that** the weight percentage of the ethanol a3) in the total weight of the cosmetic preparation a) is 50 to 95 wt.%, preferably 70 to 95 wt.% and in particular 80 to 95 wt.%.

6. The use of a product according to one of claims 1 to 5 for temporarily shaping fibers that contain keratin, in particular human hair.

7. A method for temporarily shaping fibers that contain keratin, in particular human hair, in which a cosmetic agent according to one of claims 1 to 5 is applied to the keratin fibers, and said fibers are temporarily fixed in their shape by the cosmetic agent being sprayed by means of a pressurized propellant.

## Revendications

1. Produit cosmétique comprenant un agent cosmétique destiné à la mise en forme temporaire de fibres kératiniques, comprenant
a) une préparation cosmétique contenant, par rapport au poids total de la préparation cosmétique,
a1) de 0,05 à 5,0 % en poids d'au moins une poudre d'oxyde métallique modifiée de manière hydrophobe choisie parmi les composés portant les désignations INCI Silica Dimethyl Silylate ;
a2) de 0,1 à 10 % en poids d'au moins un polymère filmogène ;
a3) de 40 à 95 % en poids d'éthanol ;
a4) au moins de 5 % en poids d'eau ;
b) un dispositif de distribution comportant une valve de pulvérisation ; et
c) un agent propulseur.

2. Agent cosmétique selon la revendication 1, **caractérisé en ce que** la proportion en poids de la poudre d'oxyde métallique hydrophobe modifiée a1) dans le poids total de la préparation cosmétique a) est de 0,1 à 4,0 % en poids, de préférence de 0,15 à 3,0 % en poids et en particulier de 0,2 à 2,0 % en poids.

3. Agent cosmétique selon l'une quelconque des revendications précédentes, **caractérisée en ce que** la proportion en poids du polymère filmogène a2) dans le poids total de la préparation cosmétique a) est de 0,2 à 8,0 % en poids, de préférence de 0,4 à 6,0 % en poids et en particulier de 0,5 à 5,0 % en poids.

4. Agent cosmétique selon l'une quelconque des revendications précédentes, **caractérisé en ce que** le polymère filmogène a2) est un copolymère des monomères
i) N-tert-octylacrylamide
ii) acide acrylique
iii) acide tert-butylaminoéthylméthacrylique
iv) et éventuellement d'autres monomères.

5. Agent cosmétique selon l'une quelconque des revendications précédentes, **caractérisé en ce que** la proportion en poids d'éthanol a3) dans le poids total de la préparation cosmétique a) est de 50 à 95 % en poids, de préférence de 70 à 95 % en poids et en particulier de 80 à 95 % en poids.

6. Utilisation d'un produit selon l'une quelconque des revendications 1 à 5 pour la mise en forme temporaire de fibres kératiniques, en particulier de cheveux humains.

7. Procédé de mise en forme temporaire de fibres kératiniques, en particulier de cheveux humains, dans lequel les fibres kératiniques sont recouvertes d'un agent cosmétique selon l'une quelconque des revendications 1 à 5 et sont fixées temporairement dans leur forme par pulvérisation dudit agent cosmétique au moyen d'un agent propulsif sous pression.
